# EUROPEAN PATENT SPECIFICATION

(11) **EP 2 179 758 B1**
(45) Date of publication and mention of the grant of the patent: **11.09.2013**
(21) Application number: 09172229.8
(22) Date of filing: 05.10.2009
(51) Int. Cl.: A61M 5/178, A61M 5/20

(54) **Device and apparatus for fractionation and infusion of radioactive drugs**
Vorrichtung und Apparat zur Fraktionierung und Infusion eines radioaktiven Arzneimittels
Dispositif et appareil pour fractionnement et perfusion d'un médicament radioactif

(30) Priority: 10.10.2008 IT FI20080193
(43) Date of publication of application: 28.04.2010
(73) Proprietor: Tema Sinergie S.r.l., 48018 Faenza RA (IT)
(72) Inventor: Piancastelli, Stefano, I-48014, Castel Bolognese (RA) (IT)
(74) Representative: Nesti, Antonio

(56) References cited:
- WO-A-03/045546
- WO-A-2008/037939
- WO-A-2008/085948

## Description

### Field of the invention

The invention relates to an apparatus for the operations of fractionation and infusion of radioactive drugs (radiopharmaceuticals), of the type used for example in nuclear medicine.

### Background art

At the current state of the art, there are known on the market automatic syringe fractionators for radioactive drugs, which, starting from a multidose reservoir or "vial", automatically fill the syringes for injection of the drugs into the patients.

There moreover exist automatic infusors that automate the process of injection into the patient using the syringe already subjected to fractionation and transferred to the infusion station.

From WO2008085948 a radiopharmaceutical injection system it is known, including a radiation shielding and a controller interconnected with an injector which may be configured to automatically control the injector in response to a signal from a biometric sensor.

Fractionators and infusors normally respond to various technical and normative requirements and have so far always been envisaged as separate machines of the more general process that on the basis of the medical prescription administers to the patient a personal, exact, and verified dose.

Also for this reason, the apparatuses currently on the market and that are able to fill the syringes and measure the dose of radioactive drug transferred into the syringe require for their operation sets of tubes, connectors, needles, valves and collection flasks (hereinafter referred to as kits), which are complex and costly, in order to enable the operators first to fill and then handle the syringes filled with the safety required by the standards.

In addition, to guarantee the necessary sterility of the process, each fractionation kit can be used for fractionation of just one vial. It is hence necessary to mount on the fractionator, for each new vial, a kit of a different supply lot (e.g. from "lot 1 of drug 1" to "lot 2 of drug 1").

Furthermore, the replacement of the kit is necessary whenever it were necessary to change the compound to be fractionated (e.g. from "drug 1" to "drug 2"), a case that occurs frequently given that it is hardly ever possible to prepare the syringes "in series" (first all the syringes of "drug 1" then all the syringes of "drug 2"), whereas much more frequently there are required syringes "in parallel" (syringes of different drugs in alternating sequences).

A considerable drawback is hence that each replacement of the kit presupposes costs and, in the case of a change of the drug to be fractionated, exposure to radiation for the operator, which basically renders necessary the acquisition of a dedicated fractionator (and frequently of a corresponding shielded cell) for each type of drug, or else, alternatively, recourse to manual fractionation.

On the other hand, the increase of the syringes per day produced in the departments of nuclear medicine leads to the need for automatic fractionation even for low-energy drugs traditionally fractionated manually.

### Summary of invention

There is consequently felt the need on the market for an apparatus that minimizes the costs of the process of fractionation, in particular the costs due to the use of the kits associated thereto.

For these and further technical purposes and advantages there is now proposed according to the invention a device in accordance with at least one of the annexed Claims 1 to 7.

The advantages obtained according to the invention consist basically in the fact that it is possible to combine in a single device the basic functions of syringe fractionation and infusion.

A second advantage is that the device according to the invention is of an automatic and programmable type, possibly with remote-control interface and enables automatic injection of the dose in the maximum safety for both the operator and the patient.

A further advantage is that the device is made of easily portable dimensions to reduce to a minimum the disposable components associated to each infusion operation.

According to a second aspect of the invention, there has been devised an apparatus for dispensing radioactive drugs that can be associated preferably to the fractionator/infusor device referred to above.

Currently known fractionation machines normally require a sterile and shielded environment within which to start up the procedure of fractionation of the liquids contained in the vials and of filling of the syringes with the doses associated to the various patients.

A drawback of these known machines is their complexity and the need to shield a relatively extensive area where fractionation takes place.

There is hence felt the need for a fractionation machine and of a corresponding method of implementation, which is simple to provide, economically advantageous to use even with repeated changes of the fractionated liquid and offers guarantees of shielding for the operator and of sterility for the operations of fractionation.

Said advantages have been achieved with an apparatus and a method according to one or more of Claims 8-11.

### List of drawings

These and further advantages will be better understood by any person skilled in the branch from the ensuing description and from the annexed drawings, provided as non-limiting example, wherein:
- Figure 1 shows a front view of the device according to the invention;
- Figure 1A shows the device of Figure 1 in the cross section A-A;
- Figure 2 shows a cross-sectional view of a preferred embodiment of a device according to the invention;
- Figure 3 shows the device of Figure 1 in perspective view;
- Figure 3A shows a device according to the invention in exploded view;
- Figure 4 shows an infusion station according to the present invention;
- Figure 5 shows a disposable infusion kit; and
- Figures 6 and 6a show an infusor according to the invention in exploded view and in the cross section A-A.

### Detailed description

With reference to the attached drawings, an infusor device 1 according to the invention comprises a section 14 containing an electronically controlled double-acting actuator for bi-directional displacement along the longitudinal axis 27 of a stem 17 coming out of the section 14.

The section 14 can then be provided with one or more manually operated pushbuttons 24, for example for operations of switching-on/switching-off and setting of the parameters for displacement of the stem, and with a possible display 25.

There may moreover be provided an interface 28 for connection to a control unit U, operatively connected to the section 14 in wired or preferably wireless mode.

The section 14 is joined by means of a coupling 15, preferably a removable fast coupling, to a cylindrical flange 2 preferably made of shielding material, which defines an internal cavity 29 and which is in turn constrained by coupling means 16, for example a bayonet fast coupling, with a hollow cylindrical body 3 made of shielding material.

The coupling 16 can moreover envisage spring-operated pins 34, which can move in hide-away fashion inside the body 3 and can be inserted in a removable way in respective reference slots made in the flange 2.

The flange 2 and the body 3 can moreover be constrained in position by means of a manually accessible clamping/release pushbutton 33.

Obtained within the body 3 is a cavity 30 in continuity with respect to the cavity 29 of the flange 2, in which there can be inserted a syringe 5 provided with a needle 23, of a type in itself known, projecting from an end hole 31 of the body 3.

Within the cavity 30 of the body 3 a cylindrical pusher 4, preferably made of a radiation-shielding material, is able to slide.

The pusher 4 is joined to one end to the stem 17 by means of a first mechanical connection 26, and to the opposite end, by means of a mechanical coupling 18, for example by interference fit, to a liquid-tight mobile piston 6 inside the syringe 5.

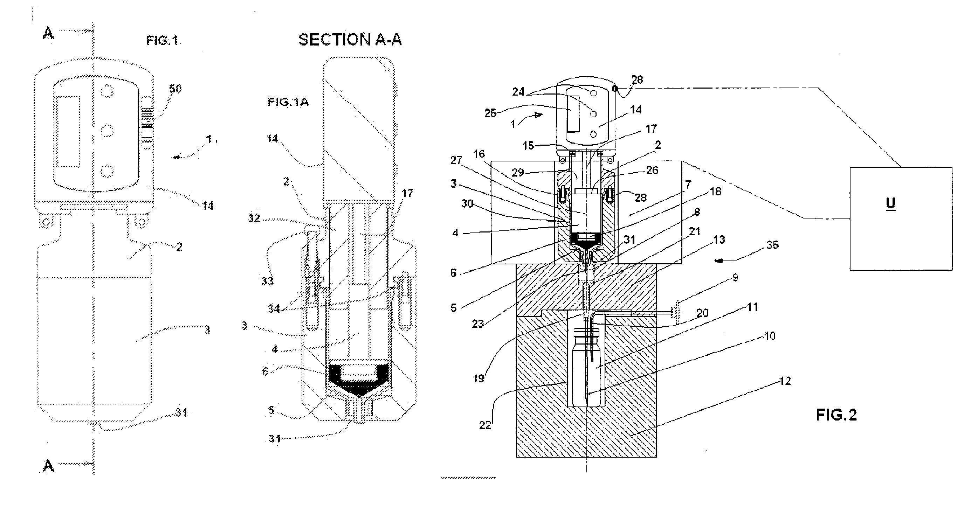

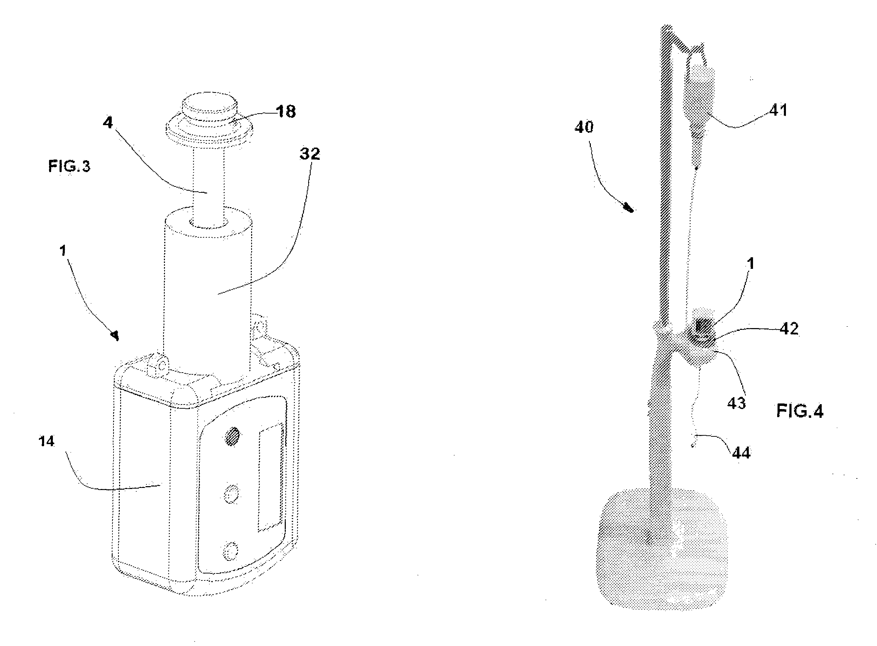

In the embodiment illustrated in Figures 1 and 3, the pusher 4 and the stem 17 are able to slide within a hollow cylindrical shielding 32, which is fixed to the section 14 and can be inserted precisely into the cavity 29 of the flange 2 and preferably projects also within the body 3 and the syringe 5.

Advantageously, the device 1 consequently enables uniting in a single machine the functions of fractionation syringe and infusion and of automatic injection of the dose to the patient.

Furthermore, the dose of radioactive liquid inserted in the syringe is completely shielded both laterally, thanks to the body 3, and axially, thanks to the pusher 4 and/or the shielding 32, and, during transition from fractionation to infusion, by the plug T (visible in figure 3A), which is also made of radiation-shielding material.

Advantageously, the device 1 at least at the moment of filling of the syringe 5 can be associated to a recognition code 50, for example a bar code or a magnetic code applied to the unit 14 or to the body 3, for associating safely the dose being fractionated with the patient associated to said dose.

Figure 2 moreover illustrates an apparatus 35 for fractionation in syringes 5 of radioactive liquid contained in a vial 11.

The apparatus 35 can comprise a hollow dose calibrator 7 connected to the control unit 14, within which the radiation-shielding body 3 of the device 1 can be inserted.

The calibrator 7 is set on top of a shielding body 12 with a hollow seat 22 for housing the vial 11. Preferably, between the shielding body 12 and the calibrator 7 there can be set a shielding plug 13, made in which is a hole 21 that defines an injection point or preferably a "no-needle" point for connection with a male Luer Lok, in which the syringe 5 projecting from the body 3 is inserted and which at the bottom communicates with the cavity 22 of the shielding body 12.

In the preferred solution illustrated in Figure 2, inserted within the hole 21, at the injection point 8, is a removable duct 19, which proceeds within the cavity 22 of the shielded body 12 and connects up to a needle 10 for drawing in the substance from the vial 11.

Moreover communicating with the inside of the vial 11 is a tubing 20, which extends as far as on the outside of the body 12 and is provided at the outer end with an air filter, preferably a 0.22-micron filter.

In operation, the control unit 14 is programmed by the operator for actuating the travel of the stem 17 during suction in order to obtain intake of the required volume of radioactive liquid into the syringe 5.

Thanks to the pusher 4 connected to the stem 17 the device 1 consequently moves upwards (in the representation of Figure 2) the piston 6, enabling filling of the syringe with the needle 5 through the needle of the vial 10 and the injection point 8.

In this step, the filter 9 enables inlet of filtered air into the vial 11 in equal volume to the liquid drawn out, preventing formation of a negative pressure inside the vial.

When the liquid has been sucked into the syringe 5, the hollow dose calibrator 7 measures the dose, i.e., the radioactive content (in millicurie) of the liquid effectively transferred into the syringe and returns the data to the control unit 14.

Advantageously, thanks to the double-acting characteristic of the actuator of the device 1, it is possible to implement also the reverse cycle, i.e., emptying part or all of the contents of the syringe 5 into the vial 11.

This characteristic hence enables, with the same device, determination of the specific activity (mCi/ml) of the liquid contained within the flask, drawing into the syringe 5 a known volume (ml) and measuring the activity (mCi) thereof with the hollow dose calibrator 7, or else proceeding to filling the syringes with the amount in volume (ml) or in activity (mCi) required.

The combination of the apparatus 35 with the device 1 enables filling of each syringe within a hot-chamber shielded cell and transfer of the syringe itself into the injection room for automatic injection of the dose, without any need for further manipulations.

Furthermore, the small dimensions of the device, allied to the high volumetric precision that can be obtained with a precision actuator, enable reduction to a minimum of the disposable components necessary, which are reduced to the injection point 8, the filter 9, the needle 10, as well as short stretches of tube of the ducts 20 and 19 and, obviously, the syringe 5 and corresponding piston 6.

Advantageously, for each different drug that is to be fractionated it thus becomes possible with a single device 1 to take from the flask desired the quantity of the radioactive drug required, in the desired sequences, simply by replacing the syringe and the needle, without at the same time subjecting the operator to any exposure.

With reference to Figure 4, there is now described an infusion station according to the invention.

Once the syringe within the device 1 has been filled with the required dose, this is inserted in a shielded seat 42 supported by a supporting collar 43 of an infusion rod 40.

Advantageously, with this solution the body 3 of the device 1 is further shielded and the patient is not exposed to any radiation during the infusion session.

The collar 43 is then accessible from the underside to enable application to the needle of the syringe 5 of the first end 45 of a tube for infusions 46 of a type in itself known, provided via connection with a connector of a no-needle type activated by a Luer Lok, or else with an injection point for a needle.

The end 45 is in turn connected to a flask for physiological solution 41 hanging from to the rod 40 and, at the free end 47 to a Luer connector with a swivel for a needle to be applied to the patient (not visible).

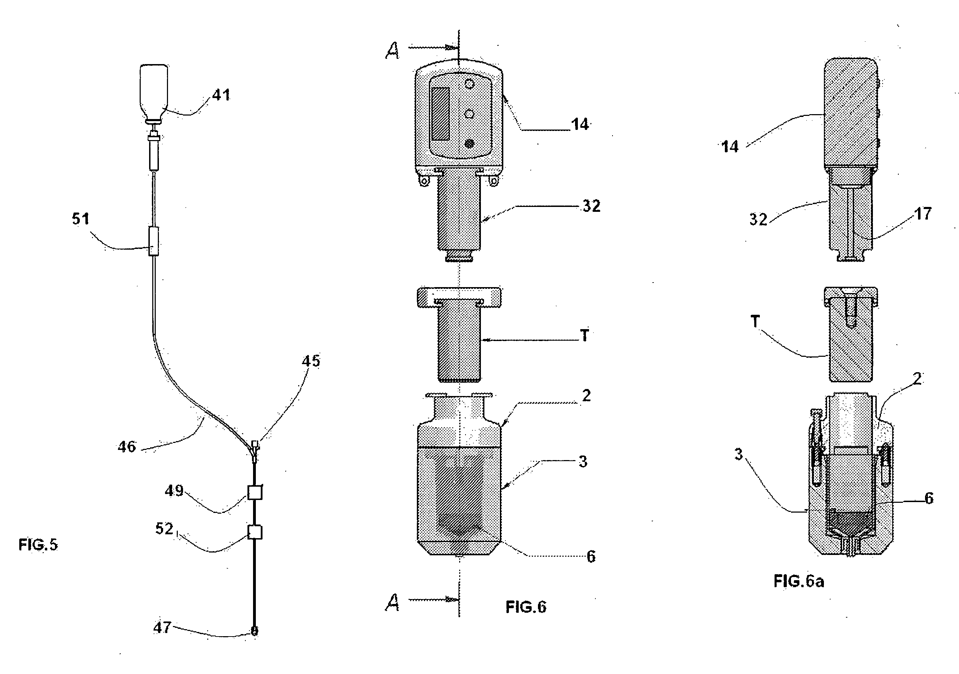

Described with reference in particular to Figure 5 is a kit of disposable components that can be used with the device according to the invention, comprising a standard flow regulator (roller) 51 set along the tube 46, a non-return valve 49, and a ventilated filter 52 with the function of separating the possible bubbles of air and getting just the liquid to flow towards the patient. Thanks to this technical solution, at the end of injection the infusor can execute a few cycles for flushing (for diluting as much as possible the liquid that inevitably remains in the unswept spaces) by drawing in physiological solution and injecting it. The non-return valve 49 ensures that in this step the infusor aspirates only physiological solution from the tube 46 and not from the end 47.

The present invention has been described according to preferred embodiments, but equivalent variants may be devised, without departing from the scope of the Invention.

## Claims

1. Portable automatic injector for radiopharmaceuticals, comprising:
- an actuation unit (14) to automatical control the displacement of a movable element (17, 4) moving along an actuation direction (27),
- a radioactive shielding body (3) provided of a cavity (30) with a first end (48) open to introduce into the shielding body (3) a syringe (5) provided with a piston (6) movable in the actuation direction (27) and with a needle (23) projecting from a hole (31) of a second end of body (3), **characterized in that** said movable element (17,4) is a bidirectional movable element and **in that** the injector further comprises
- reversible means to connect the shielding body, (3) to the actuation unit (14),
- means to link the piston (6) to the displacement of the movable element (17, 4), wherein the displacement is bidirectional.

2. Injector according to claim 1, comprising means (4) to shield said first open end (48) of body (3).

3. injector according to claim 2, wherein said movable element (17, 4) comprises a pusher (4) made of a radioactive shielding material inserted through said open end (48) and sliding within syringe (5), the pusher (4) being integral with the piston (6).

4. Injector according to claim 2, wherein said means to shield the open end (48) comprise a hollow cylinder body (32) made of a radioactive shielding material joined to the actuation unit (14), within which the cylinder body (32) said movable element (17, 4) can slide.

5. Injector according to claim 2, wherein said means to shield the open end (48) comprise a movable lid (T) of the open end (48) made of a radioactive shielding material.

6. Injector according to one of the preceding claims, comprising an interface (28) connecting said unit (14) to a control unit (U).

7. Injector according to one of the preceding claims, comprising an identifying code (50) applied to the injector to univocally associate with a user a given dose of radioactive drug filled in the syringe (5).

8. Fractioning equipment for radiopharmaceutical comprising:
an injector according to one or more of claims 1-7,
a hollow dose calibrator (7) connected to control unit (U), within which said radioactive shielding body (3) can be inserted,
a shielding body (12) having a hollow seat (22) to house a vial,
a communication tubing (8,19,10) between the syringe (5) and the vial a shielding lid (13) interposed between said calibrator (7) and said shielding body (12).

9. Equipment according to claim 8, in which said tubing (8,19,10) comprises a disposable injection point (8) and a disposable duct (19).

10. Equipment according to claim 8 or 9, comprising a duct (20) communicating with a vial (11) and extending outside of the shielding body (12), provided of an air filter at the external end.

11. Injection station of radiopharmacauticals using an injector according to one of claims 1-7, comprising a stand (40) provided of a shielded seat (42) to 5 house said body (3).

12. Station according to claim 11, in which said seat (42) is supported by a collar (43) projecting from said stand (40)

## Patentansprüche

1. Tragbares, automatisches Injektionsgerät für Radiopharmazeutika, umfassend:
- eine Betätigungseinheit (14) zur automatischen Steuerung der Verschiebung eines beweglichen Elements (17, 4), das sich längs einer Betätigungsrichtung (27) bewegt,
- ein Strahlenabschlrmgehäuse (3), gebildet aus einem Hohlraum (30) mit einem ersten Ende (48), das offen ist, um in das Abschirmgehäuse (3) eine Injektionsspritze (5) einzusetzen, die mit einem in die Betätigungsrichtung (27) bewegbaren Kolben (6) und mit einer Nadel (23), die aus einer Öffnung (31) eines zweiten Endes des Gehäuses (3) hervorsteht, versehen ist
**dadurch gekennzeichnet, dass** das bewegliche Element (17, 4) ein in zwei Richtungen bewegliches Element ist, und dadurch, dass das Injektionsgerät des Weiteren aufweist
- eine Umkehreinrichtung zum Verbinden des Abschirmgehäuses (3) mit der Betätigungseinheit (14),
- eine Einrichtung, um den Kolben (6) mit der Verschiebung des beweglichen Elements (17, 4) in Verbindung zu bringen, wobei die Verschiebung zweiseitig gerichtet ist.

2. Injektionsgerät nach Anspruch 1, umfassend eine Einrichtung (4) zum Abschirmen des ersten offenen Endes (48) des Gehäuses (3).

3. Injektionsgerät nach Anspruch 2, bei dem das bewegliche Element (17, 4) einen Schieber (4) aufweist, der aus einem Strahlenabschirmungsmaterial besteht und durch das offene Ende (48) eingesetzt wird und innerhalb der Injektionsspritze (5) gleitet, wobei der Schieber (4) mit dem Kolben (6) eine Einheit bildet.

4. Injektionsgerät nach Anspruch 2, bei dem die Einrichtung zum Abschirmen des offenen Endes (48) einen Hohlzylinderkörper (32) aufweist, der aus einem Strahlenabschirmungsmaterial besteht und mit der Betätigungseinheit (14) verbunden ist, wobei das bewegliche Element (17, 4) innerhalb dieses Zylinderkörpers (32) gleiten kann.

5. Injektionsgerät nach Anspruch 2, bei dem die Einrichtung zum Abschirmen des offenen Endes (48) einen bewegbaren Deckel (T) des offenen Endes (48), der aus einem Strahlenabschirmungsmaterial hergestellt ist, aufweist.

6. Injektionsgerät nach einem der vorhergehenden Ansprüche, umfassend eine die Einheit (14) mit einer Steuereinheit (U) verbindende Schnittstelle (28).

7. Injektionsgerät nach einem der vorhergehenden Ansprüche, umfassend einen auf das Injektionsgerät angewandten Erkennungskode (50), um eine vorgegebene Dosis eines in die Injektionsspritze (5) gefüllten radioaktiven Medikaments eindeutig einem Benutzer zuzuordnen.

8. Fraktionierausrüstung für Radiopharmazeutikum, umfassend:
ein Injektionsgerät nach einem oder mehreren der Ansprüche 1 bis 7,
eine mit der Steuereinheit (U) verbundene Hohlkörper-Dosiskalibriervorrichtung (7), in welche das Strahlenabschirmgehäuse (3) eingesetzt werden kann,
einen Abschirmungskörper (12) mit einer hohlen Aufnahme (22), um ein Medizinfläschchen unterzubringen,
Übertragungs-Röhrenmaterial (8,19, 10) zwischen der Injektionsspritze (5) und dem Medizinfläschchen,
einen Abschirmungsdeckel (13), der zwischen der Kalibriervorrichtung (7) und dem Abschirmungskörper (12) angebracht ist.

9. Ausrüstung nach Anspruch 8, bei der das Röhrenmaterial (8, 19, 10) eine wegwerfbare Einspritzung (8) und eine wegwerfbare Röhre (19) aufweist.

10. Ausrüstung nach Anspruch 8 oder 9, umfassend eine Röhre (20), die mit einem Medizinfläschchen (11) in Verbindung steht und sich außerhalb des Abschirmungskörpers (12) erstreckt und aus einem Luftfilter an dem äußeren Ende gebildet ist.

11. Injektionsstation für Radiopharmazeutika unter Verwendung eines Injektions-geräts nach einem der Ansprüche 1 bis 7, umfassend ein aus einer abgeschirmten Aufnahme (42) gebildetes Gestell (40), um das Gehäuse (3) unterzubringen.

12. Station nach Anspruch 11, bei der die Aufnahme (42) durch eine von dem Gestell (40) hervorstehende Einfassung (43) gehalten wird.

## Revendications

1. Injecteur automatique portable pour médicaments radio-pharmaceutiques comprenant :
- une unité d'actionnement (14) pour commander automatiquement le déplacement d'un élément mobile (17, 4) se déplaçant suivant une direction d'actionnement (27),
- un corps de blindage contre la radioactivité (3) pourvu d'une cavité (30) avec une première extrémité (48) ouverte pour introduire dans le corps de blindage (3) une seringue (5) munie d'un piston (6) mobile dans la direction d'actionnement (27) et d'une aiguille (23) faisant saillie à partir d'un trou (31) d'une deuxième extrémité du corps (3), **caractérisé en ce que** ledit élément mobile (17, 4) est un élément mobile bidirectionnel et **en ce que** l'injecteur comprend en outre
- des moyens réversibles pour connecter le corps de blindage (3) à l'unité d'actionnement (14),
- des moyens pour lier le piston (6) au déplacement de l'élément mobile (17, 4), dans lequel le déplacement est bidirectionnel.

2. Injecteur selon la revendication 1, comprenant des moyens (4) pour blinder ladite première extrémité ouverte (48) du corps (3).

3. Injecteur selon la revendication 2, dans lequel ledit élément mobile (17, 4) comprend un pousseur (4) constitué d'un matériau de blindage contre la radioactivité, inséré à travers ladite extrémité ouverte (48) et coulissant à l'intérieur de la seringue (5), le pousseur (4) étant d'une seule pièce avec le piston (6).

4. Injecteur selon la revendication 2, dans lequel lesdits moyens pour blinder l'extrémité ouverte (48) comprennent un corps cylindrique creux (32) constitué d'un matériau de blindage contre la radioactivité uni à l'unité d'actionnement (14), corps cylindrique (32) à l'intérieur duquel ledit élément mobile (17, 4) peut coulisser.

5. Injecteur selon la revendication 2, dans lequel lesdits moyens pour blinder l'extrémité ouverte (48) comprennent un couvercle mobile (T) de l'extrémité ouverte (48), constitué d'un matériau de blindage contre la radioactivité.

6. Injecteur selon une des revendications précédentes, comprenant une interface (28) connectant ladite unité (14) à une unité de commande (U).

7. Injecteur selon une des revendications précédentes, comprenant un code d'identification (50) appliqué à l'injecteur pour associer de manière univoque à un utilisateur une dose donnée de médicament radioactif rempli dans la seringue (5).

8. Equipement de fractionnement pour médicament radiopharmaceutique comprenant :
un injecteur selon une ou plusieurs des revendications 1 à 7,
un calibreur de dose creux (7) connecté à l'unité de commande (U), à l'intérieur duquel ledit corps de blindage contre la radioactivité (3) peut être inséré, un corps de blindage (12) ayant un siège creux (22) pour loger un flacon,
une tuyauterie de communication (8, 19, 10) entre la seringue (5) et le flacon,
un couvercle de blindage (13) interposé entre ledit calibreur (7) et ledit corps de blindage (12).

9. Equipement selon la revendication 8, dans lequel ladite tuyauterie (8, 19, 10) comprend une point d'injection jetable (8) et un conduit jetable (19).

10. Equipement selon la revendication 8 ou 9, comprenant un conduit (20) communiquant avec un flacon (11) et s'étendant à l'extérieur dudit corps de blindage (12), muni d'un filtre à air au niveau de l'extrémité externe.

11. Station d'injection de médicaments radio-pharmaceutiques utilisant un injecteur selon une des revendications 1 à 7, comprenant un pied de support (40) muni d'un siège blindé (42) pour loger ledit corps (3).

12. Station selon la revendication 11, dans laquelle ledit siège (42) est supporté par un collier (43) faisant saillie à partir dudit pied de support (40).
